# EUROPEAN PATENT APPLICATION

(11) **EP 2 230 293 A1**
(43) Date of publication of application: **22.09.2010**
(21) Application number: 08863466.2
(22) Date of filing: 18.12.2008
(51) Int. Cl.: C11C 3/10, C07C 27/02, C07C 29/76, C07C 31/22, C07C 67/03, C07C 67/56, C07C 69/24, C07C 69/58

(54) **METHOD AND APPARATUS FOR PRODUCING FATTY ACID ALKYL ESTER AND/OR GLYCERIN**

(30) Priority: 26.12.2007 JP 2007335311
(71) Applicant: Nippon Shokubai Co., Ltd., Osaka-shi, Osaka 541-0043 (JP); Research Institute Of Innovative Technology For The Earth, Kyoto 619-0292 (JP)
(72) Inventor: OKADA, Izuho, Osaka 564-8512, (JP); OKU, Tomoharu, Osaka 564-8512, (JP); HORIE, Hironori, Osaka 564-8512, (JP); NONOGUCHI, Masanori, Osaka 564-8512, (JP); TACHIBANA, Atsushi, Osaka 564-8512, (JP)
(74) Representative: Müller - Hoffmann & Partner
(86) International application number: PCT/JP2008/073067
(87) International publication number: WO 2009/081836

(57) **Abstract**

The present invention provides a method of producing a fatty acid alkyl ester and/or glycerin by bringing an oil and an alcohol into contact with each other, the method including: reacting the oil and alcohol with each other; performing solid-liquid separation for removing a solid sterol from a reaction liquid obtained in the step of reacting; and performing phase separation for separating the reaction liquid into a hydrophobic phase and a hydrophilic phase after the reaction liquid is subjected to the step of performing the solid-liquid separation, the hydrophobic phase containing the fatty acid alkyl ester, and the hydrophilic phase containing glycerin. Thereby, the present invention provides a method allowing stable continuous operation for a long time period for production of a fatty acid alkyl ester and/or glycerin.

## Description

### Technical Field

The present invention relates to a method for producing a fatty acid alkyl ester and/or glycerin, more specifically to a method and an apparatus for producing a fatty acid alkyl ester and/or glycerin which are/is suitably usable for applications to fuels, food, cosmetics, a pharmaceutical product, and the like.

### Background Art

Fatty acid alkyl esters obtained from vegetable or animal fats or oils are used as foods. In addition to such use, fatty acid alkyl esters are widely used, for example, in cosmetic and pharmaceutical fields. Note that use of a fatty acid alkyl ester as a fuel by adding it to a diesel oil has also attracted attention in recent years. Namely, such a fuel refers to a biodiesel fuel which has been developed for the purpose of reducing carbon dioxide emissions and is derived from a vegetable or animal fat or oil. The biodiesel fuel is used as a fuel which is (a) directly used as an alternative to the diesel oil or (b) added to the diesel oil at a given ratio. On the other hand, glycerin is used mainly as a material of which nitroglycerine is made. In addition to such use, glycerin is used as a material of which alkyd resin is made and is variously used, for example, in pharmaceutical, grocery, print ink and cosmetic fields.

As an example of a method for producing such a fatty acid alkyl ester and glycerin, a method has been known in which a fatty acid alkyl ester and glycerin are made by carrying out transesterification between (a) triglyceride of which a fat or oil (hereinafter, collectively referred to as oil) is mainly made and (b) an alcohol. Namely, this method includes the steps of: (a) a reaction step in which an oil and an alcohol are brought into contact with each other in the presence or absence of a catalyst; (b) a separation step in which an unreacted alcohol is removed from a reaction product obtained in the step (a); and (c) a phase separation step in which the reaction liquid is separated into (i) a hydrophobic phase in which a fatty acid alkyl ester is mainly contained and (ii) a hydrophilic phase in which glycerin is mainly contained.

A production method with a catalyst is exemplified by (i) a method in which an alkali catalyst which is a homogeneous catalyst is used and (ii) a method in which an insoluble solid catalyst which is a heterogeneous catalyst is used are disclosed (see Patent Literatures 1 through 5). In contrast, a production method without a catalyst is exemplified by a method for producing a fatty acid alkyl ester in which method an oil and an alcohol react with each other under a supercritical condition (see Patent Literature 6, for example). The method in which an insoluble solid catalyst is used is excellent among these production methods in easy purification and better product yield.
Patent Literature 1
   U.S. Patent No. 5424466
Patent Literature 2
   Japanese Patent Application Publication, Tokukai, No. 2006-225352 A (Publication Date: August 31, 2006)
Patent Literature 3
   Japanese Patent Application Publication, Tokukai, No. 2005-177722 A (Publication Date: July 7, 2005)
Patent Literature 4
   Japanese Patent Application Publication, Tokukaihei, No. 7-173103 A (Japanese Patent No. 3976800) (Publication Date: July 11, 1995)
Patent Literature 5
   Specification of French Patent Application Publication No. 2752242 A
Patent Literature 6
   Japanese Patent Application Publication, Tokukai, No. 2000-109883 A (Publication Date: April 18, 2000)

### Summary of Invention

However, the production of a fatty acid alkyl ester and/or glycerin would face a problem in which a solid matter is precipitated in a reaction liquid during the production. Namely, a solid matter may (a) accumulate in a phase separator, thereby resulting in insufficient phase separation and consequently poor product purity, (b) be attached to an inner wall of a monitor window (sight glass) provided in the phase separator, thereby making it difficult to monitor an interface level, or (c) clog piping and/or channels inside meters and gauges. In a case where such phenomena occurs, it is necessary to stop operation of a production apparatus, so as to clean a producing system. This causes a problem that a production method and a production apparatus which are described above make it impossible to realize a longtime stable continuous operation.

The present invention has been made in view of the problems. A main object of the present invention is to provide a method and an apparatus for producing a fatty acid alkyl ester and/or glycerin each of which method and apparatus allows longtime continuous operation.

In order to attain the object, the inventors of the present invention diligently carried out examination and finally accomplished the present invention by newly finding that (i) the solid matter which is precipitated in a reaction liquid is a sterol and (ii) it is possible to effectively separate the solid matter from the reaction liquid by use of an appropriate technique. The present invention has been accomplished based on the new finding and encompasses the following inventions.

A method according to the present invention is a method of producing a fatty acid alkyl ester and/or glycerin by bringing an oil and an alcohol into contact with each other, the method comprising: reacting the oil and alcohol with each other; performing solid-liquid separation for removing a solid sterol from a reaction liquid obtained in the step of reacting; and performing phase separation for separating the reaction liquid into a hydrophobic phase and a hydrophilic phase after the reaction liquid is subjected to the step of performing the solid-liquid separation, the hydrophobic phase containing the fatty acid alkyl ester, and the hydrophilic phase containing glycerin.

According to the method in accordance with the present invention in which the step of performing the solid-liquid separation is provided between the step of reacting and the step of performing the phase separation, it is possible to effectively remove the solid sterol from the reaction liquid. This can prevent the solid matter from accumulating in the phase separation apparatus, thereby causing insufficient phase separation and consequently poor product purity, or can prevent the solid matter from adhering on an internal wall of a monitoring window (sight glass) of the phase separation apparatus, thereby making it difficult to monitor an interface level or from clogging piping or flow path in meters and gauges. In the present invention, what is meant by the term "sterol" is an alcohol having a steroid moiety, and/or an ester compound of such an alcohol.

It is preferable that the method in accordance with the present invention further includes removing an unreacted alcohol from the reaction liquid obtained in the step of reacting.

The method in accordance with the present invention is preferably arranged such that the step of performing the solid-liquid separation is performed after the step of removing. According to this aspect, the removal of the unreacted alcohol diminishes solubility of sterol in the reaction liquid, thereby facilitating precipitation of the solid matter. By performing the solid-liquid separation at this stage, it is possible to remove the precipitated sterol well.

The method in accordance with the present invention is preferably arranged such that the step of performing the solid-liquid separation is carried out by passing the reaction liquid through a filter. According to this aspect, it is possible to easily remove the solid sterol.

The method in accordance with the present invention is preferably arranged such that the step of performing the solid-liquid separation is carried out by passing the reaction liquid through at least one of a plurality of the filters provided in parallel with respect to a flow of the reaction liquid.

The method in accordance with the present invention is preferably arranged such that the step of reacting is carried out under a presence of a solid catalyst. According to this aspect, it is possible to easily carry out removal of the catalyst from the reaction liquid.

An apparatus according to the present invention is an apparatus for producing a fatty acid alkyl ester and/or glycerin by bringing an oil and an alcohol into contact with each other, the apparatus comprising:
a reaction vessel for reacting the oil and alcohol with each other therein; a solid-liquid separation apparatus for removing a solid sterol from a reaction liquid; and a phase separation apparatus for separating, into a hydrophobic phase and a hydrophilic phase, the reaction liquid from which the solid sterol is removed, the hydrophobic phase containing the fatty acid alkyl ester, and the hydrophilic phase containing glycerin.

According to the apparatus in accordance with the present invention, the solid-liquid separation apparatus provided between the reaction vessel and the phase separation apparatus makes it possible to efficiently remove the solid sterol from the reaction liquid. This can prevent in advance the solid matter from accumulating in the phase separation apparatus, thereby causing insufficient phase separation and consequently poor product purity, or can prevent in advance the solid matter from adhering on an internal wall of a monitoring window (sight glass) of the phase separation apparatus, thereby making it difficult to monitor an interface level or from clogging piping or flow path in meters and gauges.

For a fuller understanding of the nature and advantages of the invention, reference should be made to the ensuing detailed description taken in conjunction with the accompanying drawings.

### Brief Description of Drawings

Fig. 1

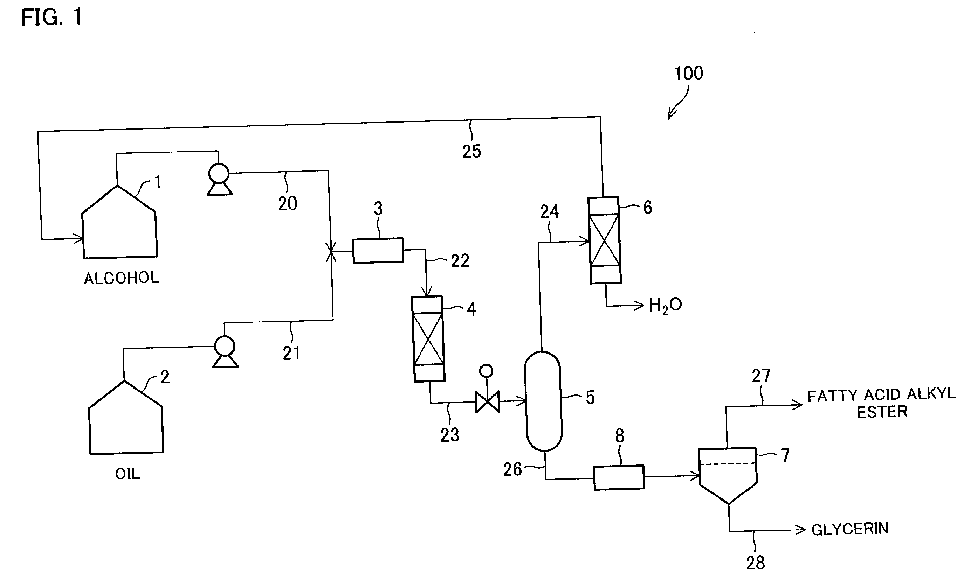

   Fig. 1 is a block diagram schematically illustrating an apparatus for a fatty acid alkyl ester and/or glycerin in accordance with a first embodiment of the present invention.

### Reference Signs List

- 1: Alcohol storage tank
- 2: Oil storage tank
- 3: Filler-system apparatus
- 4: Reaction vessel
- 5: Separation column (Separator)
- 6: Alcohol recovery column
- 7: Phase separator
- 8: Filter

### Description of Embodiments

The present embodiment discusses a method for producing a fatty acid alkyl ester and/or glycerin in accordance with the present invention. Note that "a fatty acid alkyl ester and/or glycerin" described herein is synonymous with "at least one kind of a fatty acid alkyl ester and glycerin".

This section first discusses a general reaction in which a fatty acid alkyl ester and/or glycerin are/is formed and then discusses a preferred embodiment of the present invention with reference to the drawing.

### (Reaction in which a Fatty Acid Alkyl Ester and Glycerin are Formed)

The present invention is arranged such that a fatty acid alkyl ester and glycerin are formed by a transesterification reaction of an oil and an alcohol as reaction materials. The transesterification reaction specifically refers to a reaction in which: an oil and an alcohol (methanol is exemplified in the following reaction formula) are brought into contact with each other in the presence or absence of a catalyst, whereby transesterification is caused between the oil and the alcohol. 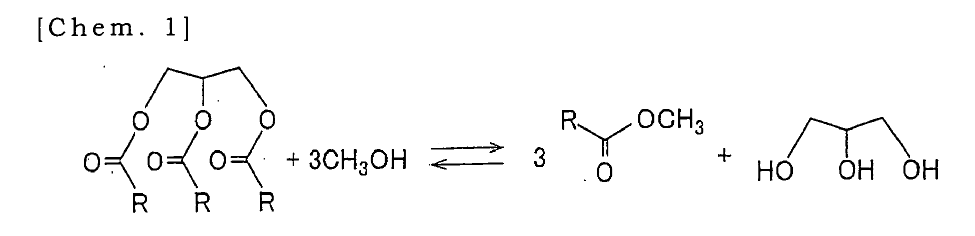 where R is (a) a C6-C22 alkyl group or (b) a C6-C22 alkenyl group which has one or more unsaturated bonds.

In the transesterification reaction, it is possible to concurrently obtain a fatty acid alkyl ester and glycerin (see the above reaction formula). Namely, it is possible to obtain, concurrently and by an industrially simple and easy method, (i) a fatty acid alkyl ester which is usable as a biodiesel fuel, a raw material for a surfactant, etc. and (ii) glycerin which is useful as a chemical material for various applications.

### [Production Apparatus]

The following description discusses a production apparatus in accordance with the present embodiment with reference to Fig. 1. Fig. 1 is a block diagram schematically illustrating the production apparatus in accordance with the present embodiment.

A production apparatus 100 for producing a fatty acid alkyl ester and/or glycerin includes an alcohol storage tank 1, an oil storage tank 2, a filler-system apparatus 3, a reaction vessel 4 (hereinafter sometimes referred to as a reaction column), a separation column (hereinafter sometimes referred to as a separator) 5, an alcohol recovery column 6, a solid-liquid separator 8, and a phase separator 7 (see Fig. 1). The alcohol storage tank 1 is a tank in which an alcohol is stored and the oil storage tank 2 is a tank in which an oil is stored. A line 20 and a line 21 are connected to the alcohol storage tank 1 and the oil storage tank 2, respectively. Each of the line 20 and the line 21 is connected to a line 22 which is connected to an upstream of the reaction vessel 4 (an end of the reaction vessel 4 to which end the reaction materials are supplied). In other words, the alcohol storage tank 1 and one end of the line 22 are connected together via the line 20 and the oil storage tank 2 and the one end of the line 22 are connected together via the line 21. Further, the other end of the line 22 (an end which is opposite to the one end of the line 22 which end is connected to each of the line 20 and the line 21) is connected to the reaction vessel 4. The line 22 is provided with the filler-system apparatus 3, via which a mixture of the alcohol and the oil is introduced into the reaction vessel 4. The reaction vessel 4 and the separation column 5 are connected together via a line 23. The separation column 5 and the alcohol recovery column 6 are connected together via a line 24. The separation column 5 and the phase separator 7 are connected together via a line 26. The line 26 is provided with the solid-liquid separator 8. A reaction liquid flowing from the separation column 5 passes through the solid-liquid separator 8, so as to be supplied to the phase separator 7. Note that the alcohol storage tank 1 and the alcohol recovery column 6 are connected together via a line 25. An alcohol recovered by the alcohol recovery column 6 returns to the alcohol storage tank 1 via the line 25, so as to be reused as the reaction material.

Note that the members included in the production apparatus are described below together with a production method.

According to the production apparatus 100 in accordance with the present embodiment, the solid-liquid separator 8 is provided on paths for the flow of a reaction liquid. The solid-liquid separator 8 can remove a solid matter well even if a solid sterol deposits in the reaction liquid. Such a solid matter would (a) accumulate in the phase separator, so as to cause insufficient phase separation and consequently a decrease in product purity, (b) be adhered to an inner wall of a monitor window (sight glass) provided in the phase separator, so as to make it difficult to monitor an interface level, or (c) clogging piping and/or channels inside meters and gauges. This will prevent stable operation of the production apparatus. However, this problem can be solved by the present embodiment with the solid-liquid separator 8 provided on the paths for the flow of the reaction liquid as described above. Namely, the production apparatus 100 can realize stable operation.

Note that the present embodiment discusses an arrangement in which one (1) solid-liquid separator 8 is provided between the reaction vessel 4 and the phase separator 7 but the present invention is not limited to this. For example, a plurality of solid-liquid separators 8 can be provided. The plurality of solid-liquid separators 8 can be provided in an identical place or respective different places on the paths for the flow of the reaction liquid. Note that in a case where a plurality of filters are provided in the identical place on the paths for the flow of the reaction liquid, it is preferable that the plurality of filters be provided in parallel with respect to the flow of the reaction liquid. Such an arrangement is advantageous to deal with a drop in filtration capability of one of the filters, because it is unnecessary to stop operation of the whole production apparatus in response to the drop in filtration capability, but it is possible to continue it by controlling a line of the production apparatus so that the reaction liquid can flow to the other of the filters. Namely, it is possible to achieve greater operational efficiency in the production apparatus.

### [Production Method]

The following description discusses a production method in accordance with the present embodiment.

A method for producing a fatty acid alkyl ester and/or glycerin in accordance with the present invention includes the steps of: (a) a reaction step in which an oil and an alcohol are brought into contact with each other so as to react with each other; (b) a solid-liquid separation step in which a solid sterol is removed from a reaction liquid obtained in the reaction step; and (c) a phase separation step in which the reaction liquid is separated into (i) a hydrophobic phase in which a fatty acid alkyl ester is contained and (ii) a hydrophilic phase in which glycerin is contained.

Furthermore, the production method according to the present embodiment preferably comprises a separation step of removing an unreacted alcohol from the reaction liquid obtained in the reaction step.

Moreover, the production method according to the present embodiment preferably comprises a removing step before the reaction step, the removing step removing, from the reaction materials including the oil and alcohol, at least one selected from the group consisting of phosphorus, phosphorus compounds, calcium, and calcium compounds. With the removing step, the production method according to the present embodiment can give the solid catalyst a longer life. In the following, the production method according to the present embodiment is explained in the order of the removing step, reaction step, separation step, the solid-liquid separation step, and the phase separation step. It should be noted that a production method using the production apparatus illustrated in Fig. 1 is explained below as a preferable embodiment of the present invention.

### <Removing Step>

The removing step is explained first here referring to Fig. 1. As described above, the removing step is a step of removing, from the reaction materials including the oil and alcohol, at least one selected from the group consisting of phosphorus, phosphorus compounds, calcium, and calcium compounds. That is, the removing step reduces a phosphorus content, phosphorus compound content, calcium content, or calcium compound content in the reaction materials.

The removing step is carried out at the filler-system apparatus 3 receiving the reaction materials from the alcohol storage tank 1 and the oil storage tank 2 via the lines 20 and 21, respectively, as illustrated in Fig. 1. The oil and alcohol are heated and pressured at the lines 20 and 21, respectively. Then, the oil and alcohol are mixed together and supplied to the filler-system apparatus 3.

The filler-system apparatus 3 is hollowed and filled inside with a filler adsorbing at least one selected from the group consisting of phosphorus, phosphorus compounds, calcium, and calcium compounds. By being introduced into the filler-system apparatus 3, phosphorus, calcium, and compounds thereof are removed from the mixture by the filler filled in the filler-system apparatus 3. The filler can be anything that adsorbs at least one selected from the group consisting of phosphorus, phosphorus compounds, calcium, and calcium compounds. Specific examples of the filler encompass silica, alumina, silica alumina, activated earth, diatom earth, titania, zirconia, ferric oxide, hydrotalcite, activated carbon, etc. Among these, silica, alumina, silica alumina, titania, zirconia, and activated carbon are preferable.

In the present Description etc., phosphorus atom concentration (hereinafter, may be referred to as phosphorus concentration simply) and calcium atom concentration (hereinafter, may be referred to as calcium concentration simply) are determined by using an inductively coupled plasma mass spectrometry (ICP-MS).

The removing step reduces the phosphorous content in the reaction materials including the oil and the alcohol to less than 2.5 ppm preferably, 2 ppm or less more preferably, and 1.5 ppm or less further preferably. The removing step reduces the calcium content in the reaction materials including the oil and the alcohol to less than 1 ppm preferably, 0.8 ppm or less more preferably, 0.5 ppm or less further preferably, and most preferably 0.2 ppm or less. By reducing the phosphorus content to less than 2.5 ppm at least and the calcium content to less than 1 ppm at least, it is possible to reduce an amount of coating of a surface of the solid catalyst with at least one selected from the group consisting of phosphorus, phosphorus compounds, calcium, and calcium compounds. As a result, it becomes possible to lower a rate of deterioration in the activity of the solid catalyst due to the coating of the solid catalyst with at least one selected from the group consisting of phosphorus, phosphorus compounds, calcium, and calcium compounds. That is, the solid catalyst can be given a longer life.

The longer life of the solid catalyst can reduce frequency of replacement of a solid catalyst whose activation is deteriorated to require the replacement, thereby reducing troublesome operation. Further, this arrangement can increase yield of the fatty acid alkyl ester and/or glycerin per unit amount of the solid catalyst. Thus, the cost of the solid catalyst per product can be lowered, thereby increasing the productivity of the fatty acid alkyl ester and/or glycerin.

In Fig. 1, the filler-system apparatus 3 is provided in the downstream of the confluence of the lines 20 and 21. However, the filler-system apparatus 3 may be provided at any point, provided that it is positioned in the upstream of the transesterification reaction. That is, the filler-system apparatus 3 may be provided on each of the lines 20 and 21. The filler-system apparatus 3 may be provided at an inlet of the reaction vessel 4 (described later) from which inlet the reaction materials are supplied into the reaction vessel 4. The filler-system apparatuses 3 may be integrated to the oil storage tank 2 and the alcohol storage tank 1, respectively. As a further alternative, the filler-system apparatus 3 may be provided in the upstream of each of the oil storage tank 2 and the alcohol storage tank 1, so that the oil storage tank 2 and the alcohol storage tank 1 are supplied respectively with an oil and alcohol with which the reaction can be carried out with phosphorus content of less than 2.5 ppm and/or calcium content of less than 1 ppm as a whole.

Moreover, as described above, the contents of phosphorus, phosphorus compounds, calcium, and calcium compounds in alcohols are negligibly small. Thus, the production method may be arranged such that the phosphorus concentration and the calcium concentration are reduced in the oil but not in the alcohol, so that the reaction materials including the oil and the alcohol have the phosphorus concentration of less than 2.5 ppm and the calcium concentration of less than 1 ppm, as a whole.

Furthermore, it is preferable that a plurality of the filler-system apparatuses 3 are provided in parallel toward a flow direction of the raw materials. Even if the filler in one of the filler-system apparatuses is being replaced, the rest of the filler-system apparatuses not under the filler replacement can continue the removal of the at least one selected from the group consisting of phosphorus, phosphorus compounds, calcium, and calcium compounds. Thus, this arrangement allows the filler replacement in the filler-system apparatuses without stopping operation of the production apparatus, that is, without stopping the production of the fatty acid alkyl ester and/or glycerin. Therefore, this arrangement can avoid a decrease in the productivity of the fatty acid alkyl ester and/or glycerin.

The removal of the at least one selected from the group consisting of phosphorus, phosphorus compounds, calcium, and calcium compounds is not limited to the use of the filler-system apparatus 3, provided that the at least one selected from the group consisting of phosphorus, phosphorus compounds, calcium, and calcium compounds can be removed. As an alternative to the use of the filler-system apparatus 3, a device for membrane separation, distillation, extraction, or the other removing means may be provided in order to carry out the removal.

The oil as the raw material has been subjected to degumming process, prior to the removing step. The degumming process removes a gumming components such as phospholipids and proteins from the oil. The degumming process is conventionally well known. More specifically, in the degumming process, water and an acid selected from the group consisting of phosphoric acid, sulfuric acid, hydrochloric acid, boric acid, and citric acid are added to oil so as to hydrate the gumming compounds and then centrifuge the hydrated gumming compounds out of the oil.

### <Reaction Step>

Next, the reaction step is explained. In the reaction step, the oil and the alcohol are brought into contact so as to cause the transesterification between them. As illustrated in Fig. 1, the reaction material with reduced phosphorus or phosphorous compound content is supplied to the reaction vessel 4 via the line 22. In the present embodiment, the reaction using the solid catalyst is described. In this case, the reaction vessel 4 is filled with the solid catalyst.

A reaction temperature, that is, a temperature of the reaction materials in the reaction vessel 4, is preferably in a range of 50 to 300°C, more preferably in a range of 70 to 290°C, and further preferably in a range of 100 to 280°C. The reaction temperature in the range can sufficiently increase a reaction rate while sufficiently preventing the alcohol from decomposing.

A reaction pressure, that is, a pressure in the reaction vessel 4, is preferably in a range of 0.1 to 10 MPa, more preferably in a range of 0.2 to 9 MPa, and further preferably in a range of 0.3 to 8 MPa. The reaction pressure in the range can sufficiently increase the reaction rate while sufficiently suppressing a side reaction. Moreover, a reaction pressure greater than 10 MPa requires a special device tolerant against high pressure, thereby requiring an extra cost for facility, etc.

Moreover, an amount of the reaction materials to be supplied to the reaction vessel 4, that is, amounts of the alcohol and the oil to be supplied to the reaction vessel 4 are preferably such that the amount of the alcohol to be supplied is preferably 1 to 30 times, more preferably 1.2 to 20 times, further preferably 1.5 to 15 times, and much further preferably 2 to 10 times greater than a theoretical requisite amount thereof required for the amount of the oil to be supplied. The oil and the alcohol can be sufficiently reacted with each other and conversion rate of the oil can be improved, when the amounts of the alcohol and the oil to be supplied are in the range. Moreover, it is possible to reduce an amount of the alcohol recovered in a first purification step (later described) and a utility cost for the alcohol recovery column 6. Thus, the production cost can be reduced.

The theoretical requisite amount of the alcohol in the present Description etc. is a molar amount of the alcohol required for a saponification value of the oil, and can be calculated out by the following equation.

Theoretical requisite amount of alcohol (g) = molecular amount of alcohol x [amount of oil (g) x saponification value (mg (KOH) / g (oil))/56100].

The reaction vessel 4 may be a batch type or a fixed-bed flow type. It is preferable that the reaction vessel 4 is a fixed-bed flow type, which does not require separation step for the catalyst. This eliminates a troublesome operation from the process, thereby making it easier to perform the industrial production.

In case where the reaction vessel 4 is a fixed-bed flow type, an average residence time of a mixture solution in the reaction vessel 4 is preferably in a range of 1 minute to 5 hours, more preferably in a range of 15 minutes to 4 hours, and further preferably in a range of 30 minutes to 2 hours. When the mixture solution is retained in the reaction vessel 4 in such an average residence time, it becomes possible to cause the alcohol and the oil to react sufficiently. Because a longer average residence time requires the reaction vessel 4 to be greater in size, the average residence time within the range is preferable in order that the reaction vessel 4 may be in a reasonable size.

Moreover, the reaction vessel 4 may be a batch-type reaction vessel. For the reaction vessel 4 of the batch type, the catalyst is introduced into the reaction materials including the oil and the alcohol. The reaction time is dependent on the catalyst volume, but usually the reaction time is preferably in a range of 15 minutes to 30 hours, and more preferably in a range of 30 minutes to 20 hours.

### <Separation Step>

Next, the separation step is described here. The separation step is a step of separating, mainly, the unreacted alcohol from a reaction liquid obtained in the reaction step. As illustrated in Fig. 1, the reaction liquid containing the fatty acid alkyl ester and glycerin obtained by passing through the reaction vessel 4 is supplied to the separation column 5 via the line 23. The reaction liquid thus obtained contains glyceride, free fatty acid, the unreacted alcohol, water, etc. in addition to the fatty acid alkyl ester and the glycerin. In the reaction liquid, an active component of the insoluble solid catalyst is preferably not more than 1000 ppm, more preferably not more than 800 ppm, further preferably not more than 600 ppm, especially preferably not more than 300 ppm, and most preferably at an undetectable level for an analyzing device.

The unreacted raw material alcohol contained in the reaction liquid can be distilled off by adjusting a pressure and a temperature of the separation column. The pressure in the separation column is not particularly limited, provided that the unreacted raw material alcohol can be separated from the products, namely, the fatty acid alkyl ester and glycerin. It is preferable that the pressure in the separation column is lower than that in the reaction vessel 4. Moreover, the temperature of the separation column 5 is preferably adjusted in consideration of boiling points of the alcohol used as the raw material, and of the fatty acid alkyl ester and glycerin obtained as the products. More specifically, the temperature of the separation column 5 is at the boiling point of the unreacted raw material alcohol under the pressure inside the separation column, and is not higher than lower one of the boiling points of the fatty acid alkyl ester and glycerin obtained as the products.

It is preferable that the alcohol thus distilled off is reused as the raw material after being subjected to water removal.

In case the alcohol thus distilled off is reused as the raw material, the alcohol, including water, thus distilled off is supplied to the alcohol recovery column 6 via the line 24, as illustrated in Fig. 1. The alcohol, including water, thus distilled off is subjected to the water removal to separate them by a conventionally well-known method such as distillation, membrane filtration, adsorption, or the like. The alcohol thus separated via the alcohol recovery column 6 is returned to the alcohol storage tank via the line 25, so that it can be reused as the raw material.

In case where water is contained in the reaction materials, the reaction step also undergoes a hydrolysis reaction of the ester obtained in the reaction step. Therefore, in case where the alcohol separated via the alcohol recovery column 6 is reused as the raw materials, it is preferable that the alcohol to be reused is lowered in its moisture content down to preferably 0.001 to 5 %, more preferably 0.005 to 3 %, and further preferable to 0.01 to 1.5%. With this arrangement, it is possible to avoid a low yield of the fatty acid alkyl ester by preventing the hydrolysis of the fatty acid alkyl ester from proceeding in the reaction step.

Moreover, the separation column 5 may also function as an alcohol recovery column, thereby allowing to omit the alcohol recovery column 6 in the process.

### <Solid-Liquid Separation Step>

Next, the solid-liquid separation step is described. The solid-liquid separation step is a step of removing a solid matter from the reaction liquid obtained in the reaction step or from the reaction liquid having been subjected to the separation step by which the content of the unreacted raw material alcohol is lowered. The solid matter is mainly a sterol. In the present Description, the sterol is an alcohol and/or an ester compound thereof, the alcohol having a steroid moiety. It is known that such an alcohol and/or an ester compound thereof is present in animal or vegetable oil etc. as a minor component. Moreover, the "removal of the solid matter (mainly a sterol) is not limited to perfect elimination of the solid matter from the reaction liquid but may be partial removal thereof from the reaction liquid.

The solid-liquid separation step passes the reaction liquid through a solid-liquid separation device, the reaction liquid being obtained in the reaction step or having been subjected to the separation step thereby being lowered in its content of the unreacted raw material alcohol. The solid-liquid separation device 8 is not particularly limited, provided that the sterol precipitated in the reaction liquid can be removed from the reaction liquid by the solid-liquid separation device 8. For example, the solid-liquid separation device 8 may be a filter, a centrifugal device, a sedimentation tank, etc. Among them, it is preferable to use a filter as the solid-liquid separation device 8, in view of better energy efficiency, device scale, etc. The filter may be: a filtering medium filter such as screen type (membrane filter, micro strainer, etc.), deep-bed filter (membrane filter, particle layer filtration, fiber layer filtration, etc.); a cake filter such as vacuum-type or pressured type filter (drum type, disc type, horizontal type, leaf-shape type, candle-shape type, etc), filter press, roll (belt) press, screw press. The filtering medium is more preferable in consideration of the concentration of the solid matter in the reaction liquid, particle diameter, facility cost, and running cost. As to a filtration accuracy of the filter, the filter is only required to be able to remove the solid matter to the extent that the solid-liquid separation step is effective. So, the filter is not particularly limited in terms of the filtration accuracy. In consideration of particle diameters of the solid matter, the filtration accuracy is preferably 100 µm or less, more preferably 50 µm or less, further preferably 10 µm or less, and especially preferably 5 µm or less. Moreover, in order that the reaction liquid may pass through the filter at a sufficient flow rate, the filtrate accuracy is preferably 0.1 µm or more, and more preferably 1 µm or more. In the present Description, the "filtration accuracy" means that the filter can capture 99.9% of the solid matter in a short diameter equal to or greater than the value. That is, by using a filter with a filtration accuracy of 5 µm, it is possible to remove 99.9% or more of the solid matter in a short diameter of 5 µm or greater from the reaction liquid supplied to the solid-liquid separation step.

Moreover, the solid-liquid separation step is carried out preferably after the separation step. As a result of the separation of the unreacted raw material alcohol from the reaction liquid by the separation step, the sterol in the reaction liquid is lowered in solubility, thereby making it easier for the solid sterol to precipitate. Thus, by performing the separation step before the solid-liquid separation step, the removal of the solid sterol becomes more effective. Moreover, the solubility of the sterol is also temperature dependent. For more effective sterol removal, the solid-liquid separation step is carried out preferably at 100°C or lower, more preferably at 80°C or lower, and further preferably at 60°C or lower. As to a lower limit of the temperature, the solid-liquid separation step is carried out preferably at 30°C or higher, and more the preferably at 40°C or higher, in order to carry out the solid-liquid separation step at a temperature at which an intermediate glyceride in the reaction liquid will not precipitate out.

### <Phase Separation Step>

Next, the reaction liquid having been treated with the solid-liquid separation apparatus 8 is fed on the phase separation apparatus 7 via the line 26. The reaction liquid is phase-separated into a hydrophobic phase and hydrophilic phase by the phase separation apparatus 7. The phase separation apparatus may be a continuous phase separation tank, in which the reaction liquid is continuously supplied and each phase is taken out from an upper side and a lower side of the continuous phase separation tank respectively by an overflow method. The continuous phase separation tank is employed with monitoring an interface between the upper and lower phases, and with adjusting a feeding rate of the reaction liquid and a level of the interface.

The production method according to the present embodiment is more advantageous when such a continuous phase separation tank is employed. At a middle height thereof, the continuous phase separation tank has a monitoring window (so-called "sight glass") for monitoring the interface between the upper and lower phases. If the reaction liquid introduced in the phase separation apparatus contains a solid sterol, the solid sterol might adhere to the monitoring window, thereby making it difficult to monitor the interface between the upper and lower phases. In this case, the level of the interface and a state of the phase separation cannot be monitored. This will cause problems to process management. For example, this would lead to a delay of detecting a trouble such as insufficient separation. Furthermore, the solid matter would accumulate in the phase separation tank, thereby causing insufficient separation of the upper and lower phase. The insufficient separation would result in mixing the lower phase into an overflow liquid of the upper phase or vice versa, thereby deteriorating purity of the product. However, the present invention can avoid this problem, because the solid sterol is removed before the phase separation step. Thus, the present invention allows the phase separation apparatus to operate stably for a long time.

The upper phase thus separated out is transferred via the line 27 and collected as the fatty acid alkyl ester, whereas the lower phase thus separated is transferred via the line 28 and collected as glycerin. The fatty acid alkyl ester and glycerin thus obtained may be purified by distillation, fractional distillation, or the like, in order to be more suitable for usage. The purification of the fatty acid alkyl ester and glycerin may be carried out by a conventionally known method.

Moreover, the production method and production apparatus according to the present invention may comprise or carry out a multi-staged reaction step and may be preferably embodied as repeating the aforementioned steps plural times.

The transesterification is an equilibrium reaction, so that a single-staged reaction would result in residual glyceride (reaction intermediate) in the hydrophobic phase obtained via the line 27. If such a glyceride remains therein, it is preferable that the glyceride is further reacted by a conventionally known method so as to remove the glyceride therefrom. That is, it is preferable that the fatty acid alkyl ester collected from the line 27 is used as the raw material again so that the transesterification with the alcohol is carried in two stages. With this method, it is possible to lower an amount of the residual intermediate glyceride, thereby further improving the targeted fatty acid alkyl ester in purity (quality).

### [Reaction Raw materials and Solid Catalyst]

Next, the reaction materials and solid catalyst usable in the present invention are described.

### <Solid Catalyst>

In the present description, the "solid catalyst" is a compound having a catalytic effect in the transesterification with hardly dissolved in the reaction liquid containing the raw materials and/or the reaction products.

The solid catalyst suitably usable in the present invention is preferably an insoluble solid catalyst insoluble in the oil and alcohol as the raw materials and the fatty acid alkyl ester and glycerin as the reaction products. The term "insoluble" used for the solid catalyst in the present Description etc. means that in the reaction liquid after the transesterification, the active component (e.g., active metal component) of the solid catalyst is not more than 1000ppm, preferably not more than 800ppm, more preferably not more than 600ppm, further preferably not more than 300ppm, and most preferably at an undetectable level for an analyzing device.

If the active component of the solid catalyst in the reaction liquid is kept not to be greater than 1000 ppm in the reaction liquid, it is possible to sufficiently suppress reverse reaction in the transesterification of the alcohol and the oil. Moreover, it is possible to omit a step of removing the active component from the reaction liquid in which it is dissolved.

The concentration of the active component of the solid catalyst in the reaction liquid (i.e., an amount of the active component dissolved in the reaction liquid) can be measured by using X-ray fluorescence analysis (XRF). XRF can analyze the post-reaction reaction liquid as it is in the form of solution. Moreover, in order to measure a smaller amount of the active component dissolved in the reaction liquid, an inductively coupled plasma atomic emission spectroscopy (ICP-AES) may be adopted for the analysis.

The solid catalyst suitably usable in the present invention is not particularly limited, provided that it is insoluble in the oil and alcohol as the raw materials, and the reaction products (fatty acid alkyl ester and glycerin), etc. However, it is preferable that the solid catalyst is easy to remove from a reaction system after the transesterification.

Moreover, the solid catalyst is preferably a catalyst catalytic to esterification of a free fatty acid included in the oil, that is, a catalyst catalytic to both transesterification of glyceride contained in the oil and esterification of the free fatty acid. This arrangement makes it possible to carry out the transesterification of the alcohol and oil while esterifying the free fatty acid, even if the oil as the raw material contains the free fatty acid. This makes it unnecessary to perform the esterification separately from the transesterification, thereby simplifying the process and improving the yield of the fatty acid alkyl ester to obtain.

More specifically, the solid catalyst as described above is preferably an alkali metal-containing compound, alkali earth metal containing compound, aluminum-containing compound, silica-containing compound, titanium-containing compound, vanadium-containing compound, chrome-containing compound, manganese-containing compound, iron-containing compound, cobalt-containing compound, nickel-containing compound, copper-containing compound, zinc-containing compound, zirconium-containing compound, niobium-containing compound, molybdenum-containing compound, tin-containing compound, rare earth-containing compound, tungsten-containing compound, lead-containing compound, bismuth-containing compound, or ion exchange resin.

The compound is not particularly limited, provided that it has the essential component. For example, the compound is preferably an oxide, sulfate, phosphate, cyanide, halide, or complex, as a sole compound, a mixture, or a composite. Among them, an oxide or cyanide as a sole compound, a mixture thereof, or a composite thereof is more preferable. More specifically, an aluminum oxide, titanium oxide, manganese oxide, zinc oxide, zirconium oxide, and a mixture and/or a composite thereof with each other or with another metal is more preferable, and zinc cyanide, iron cyanide, cobalt cyanide, and a mixture and/or a composite thereof with each other or with another metal is more preferable. These compounds in these forms may be supported by or immobilized on a carrier. Examples of the carrier encompass silica, alumina, silica·alumina, various types of zeolite, activated carbon, diatom earth, zirconium oxide, titanium oxide, tin oxide, lead oxide, and the like.

Moreover, examples of the ion exchange resin encompass an anion exchange resin. Examples of the anion exchange resin encompass strong basic anion resin, and weak basic anion resin, etc. When the anion exchange resin is classified in terms of a degree of cross linkage or a degree of porosity, the examples of the anion exchange resin encompass gel type, porous type, and high porous type, etc.

### <Oils>

The oils usable in the present invention are described below.

The oil can be any oil or fat that contains a fatty acid ester of glycerin and can be a raw material for the fatty acid alkyl ester and/or glycerin, in combination with the alcohol. Thus, the oil is not particularly limited and may be an material that is generally referred to as "oil or fat" and contains a fatty acid ester of glycerin. In generally, it is preferable that an oil which mainly contains a triglyceride (triester of glycerin and a higher fatty acid) and which contains diglyceride, monoglyceride, free fatty acid, and other accessory component in a small amount. However, the oil may be a fatty acid ester of glycerin such as triolein or tripalmitin.

Specific examples of such an oil encompass vegetable oils such as coconut oil, canola oil, sesame oil, soybean oil, corn oil, sunflower seed oil, palm oil, palm kernel oil, copra oil, safflower oil, linseed oil, cotton oil, tung oil, castor oil, etc., animal oils such as beef tallow, lard, fish oil, whale oil, etc., used editable oils (edible oil waste) of various type, and the like. Among these, palm oil is more preferable because it is low in phospholipid content. These oil may be used solely or in combination.

### <Alcohol>

Finally, alcohols usable in the present invention is described below. In the present Description, the "alcohol" is a collective term for hydrocarbons with a hydrogen(s) being substituted with a hydroxyl group(s).

In order to produce a biodiesel fuel, the alcohol is preferably a C1 to C6 alcohol, and more preferably a C1 to C3 alcohol. Examples of the C1 to C6 alcohol encompass methanol, ethanol, propanol, isopropyl alcohols, 1-butanol, 2-butanol, t-butyl alcohols, 1-pentanol, 3-pentanol, 1-hexanol, and 2-hexanol, etc. Among them, methanol and ethanol are preferable. Moreover, these alcohols may be used solely or in combination.

In order to produce raw materials for an edible oil, cosmetics or medicines, the alcohol is preferably a polyol. Examples of the polyol encompass ethylene glycol, propylene glycol, glycerin, pentaerythritol, and sorbitol, etc. Among them, glycerin is preferable. Moreover, these alcohols may be used solely or in combination.

In case the alcohol is a polyol, the method according to the present invention for producing the fatty acid alkyl ester may be read as a method for producing a glyceride, and can be applied to glyceride production.

The method according to the present invention for producing fatty acid alkyl ester and/or glycerin may be arranged such that a component other than the oil, alcohol, and solid catalyst is present in a small amount.

The present invention is not limited to the description of the embodiments above, but may be altered by a skilled person within the scope of the claims. An embodiment based on a proper combination of technical means disclosed in different embodiments is encompassed in the technical scope of the present invention.

### Examples

In the following, Examples of the method according to the present invention for producing fatty acid alkyl ester and/or glycerin is described. It should be noted that the Examples are merely to illustrate a preferable mode of the present invention and not to limit the present invention in any extent.

### [Example 1]

### <Preparation of Solid Catalyst>

Manganese carbonate (30 parts), anatase-type titanium oxide (19 parts), and alkyl cellulose (methollose 90SH-15000, produced by Shin-Etsu Chemical Co., Ltd.; 1 part) were mixed well. To mixture powder thus obtained, water of 19 parts was added uniformly. The addition of the water was carried out by adding plural portions of the amount of water separately. Then, the mixture was well blended. Then, by using a wet granulating extruder (Dome Gran, produced by Fuji Paudal Co., Ltd), the mixture was extruded via a die having pore size of 0.4 mm in diameter. The extruded was dried for one day and one night at 120°C, and then sheared into about 5 mm in length by a pulverizing device (sample mill, produced by Fuji Paudal Co., Ltd.). After that, it was calcinated at 1000°C under air atmosphere for 5 hours, thereby obtaining a catalyst MnTiO₃.

The solid catalyst (15mL) thus obtained was filled in a SUS-316-made straight reaction tube (reaction vessel 4) having an internal diameter of 10mm and a length of 210mm. At an outlet thereof, the reaction tube was controllable in pressure by being provided with a filter, an air-cooling type cooling tube, and a back pressure valve, where the filter and back pressure valve are provided at an outlet of the reaction tube via the air-cooling type cooling tube.

### <Oil and Alcohol>

The oil was palm oil, and the alcohol was methanol herein. The palm oil was a degummed and purified palm oil.

### <Reaction Step>

From a methanol storage tank (alcohol storage tank 1) and a palm oil storage tank (oil storage tank 2), in which palm oil and methanol were respectively stored, palm oil (flow rate: 0.12ml/min) and methanol (flow rate: 0.13ml/min) were continuously supplied by using a high-pressure accurate metering pump. The reaction tube was placed in an heating oven in which oven temperature was set at 200°C. The pressure of the reaction tube was adjusted to 5 MPa by using the back pressure valve at the outlet of the reaction tube. By using a line heater, a reaction liquid was kept at 200°C until the pressure was released by the back pressure valve.

### <Separation Step>

Then, the reaction liquid, which was partially vaporized by releasing of the back pressure valve, was introduced into a stainless-steel (SUS 316) flash drum (separation column 5) having a length of 360mm and an internal diameter of 80mm, and being provided with a liquid reservoir of 200mL volume at a bottom of the column. The drum was kept at 90°C by a ribbon heater wrapping around the drum. Methanol gas was distilled out from a top of the column of the drum, and then collected by being liquefied by a condenser. In the liquid reservoir at the bottom of the column, the reaction liquid (column-bottom liquid) was vigorously stirred with a rotor and heated at 150°C while being kept at 100 mL in the liquid reservoir by being continuously pumped out by a metering pump.

### <Solid-Liquid Separation Step>

The reaction liquid was cooled down to 50°C by a cooling device, and then filtered by a filler (filtering medium, Polynet produced by 3M company) having a filtration accuracy of 5µm, so as to separate and remove a solid sterol from the reaction liquid. In this way, 0.03 mass% of the solid sterol was separated and collected with respect to the purified palm oil supplied as the raw material.

### <Phase Separation Step>

The reaction liquid (filtrate) obtained via the filtration was continuously fed into a continuous phase separation tank (corresponding to phase separation apparatuses 7) of 17mm in width, 100mm in depth, and 250mm in length. An upper phase (hydrophobic phase) and a lower phase (hydrophilic phase) were respectively collected from the continuous separation tank by overflow method. From the upper phase, fatty acid methyl ester (88% pure) was obtained at a flow rate of 0.11ml/min, and from the lower phase, glycerin (96% pure) was obtained at a flow rate of 0.01ml/min. No adhesion of the solid sterol was observed in the phase separation apparatus and outlet pipes of the phase separation apparatus. Further, continuous operation was successfully carried out stably for 2000 hours or longer.

### [Comparative Example 1]

A reaction was carried out in the same manner as in Example 1, except that the filtration (Solid-Liquid separation) was not carried out. An outlet line of phase separation apparatus was clogged with the solid sterol, thereby failing to perform the continuous operation stably for 70 hours or longer. Moreover, the solid sterol adhered to an internal wall and a sight glass of the phase separation apparatus, thereby making it difficult to visually monitor a state of the phase separation and a level of an interface between the upper and lower phases. Roughly every 200 hours, the phase separation apparatus had to be dissembled and cleaned.

From Example 1 and Comparative Example 1, it was confirmed that the production method according to the present invention makes it possible to perform long-term continuous operation.

According to the production method of the present invention, it is possible to remove the solid sterol from the reaction liquid between the reaction step in which the oil is brought into contact with the alcohol and the phase separation of the reaction liquid. This can prevent accumulation of a solid matter in the phase separation apparatus thereby causing insufficient phase separation and consequently low product purity, or can prevent clogging of a pipe or a flow path inside meters etc. with the solid matter. Further, in case where the separation interface is visually monitor via a monitoring window (sight glass) of the phase separation apparatus, this arrangement can prevent adhesion of sterol to the internal wall of the monitoring window (sight glass), so that sterol adhered thereto will not make it difficult to judge the interface level.

Note that a production apparatus in accordance with the present invention (i) makes it possible to carry out a production method as described above and (ii) allows long-term stable continuous operation.

The embodiments and concrete examples of implementation discussed in the aforementioned detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### Industrial Applicability

A fatty acid alkyl ester and/or glycerin which (i) are/is produced by use of a production method in accordance with the present invention and (ii) have/has a high quality are/is usable for applications to a fuel, food, cosmetics, a pharmaceutical product, and the like. In particular, a fuel in which a fatty acid alkyl ester is used is suitably usable as a new fuel in which carbon dioxide emissions which may cause global warming are reduced.

## Claims

1. A method of producing a fatty acid alkyl ester and/or glycerin by bringing an oil and an alcohol into contact with each other, the method comprising:
reacting the oil and alcohol with each other;
performing solid-liquid separation for removing a solid sterol from a reaction liquid obtained in the step of reacting; and
performing phase separation for separating the reaction liquid into a hydrophobic phase and a hydrophilic phase after the reaction liquid is subjected to the step of performing the solid-liquid separation, the hydrophobic phase containing the fatty acid alkyl ester, and the hydrophilic phase containing glycerin.

2. The method as set forth in claim 1, comprising:
removing an unreacted alcohol from the reaction liquid obtained in the step of reacting.

3. The method as set forth in claim 2, wherein:
the step of performing the solid-liquid separation is performed after the step of removing.

4. The method as set forth in any one of claims 1 to 3, wherein:
the step of performing the solid-liquid separation is carried out by passing the reaction liquid through a filter.

5. The method as set forth in claim 4, wherein:
the step of performing the solid-liquid separation is carried out by passing the reaction liquid through at least one of a plurality of the filters provided in parallel toward a flow of the reaction liquid.

6. The method as set forth in any one of claims 1 to 5, wherein:
the step of reacting is carried out under a presence of a solid catalyst.

7. An apparatus for producing a fatty acid alkyl ester and/or glycerin by bringing an oil and an alcohol into contact with each other, the apparatus comprising:
a reaction vessel for reacting the oil and alcohol with each other therein;
a solid-liquid separation apparatus for removing a solid sterol from a reaction liquid; and
a phase separation apparatus for separating, into a hydrophobic phase and a hydrophilic phase, the reaction liquid from which the solid sterol is removed, the hydrophobic phase containing the fatty acid alkyl ester, and the hydrophilic phase containing glycerin.
